(19) 

**Europäisches Patentamt**
**European Patent Office**
**Office européen des brevets**

(11) **EP 4 304 509 B1**

(12) **EUROPEAN PATENT SPECIFICATION**

(45) Date of publication and mention
of the grant of the patent:
**14.05.2025 Bulletin 2025/20**

(21) Application number: **22713203.2**

(22) Date of filing: **10.03.2022**

(51) International Patent Classification (IPC):
*A61B 34/00* (2016.01)  *A61B 34/37* (2016.01)

(52) Cooperative Patent Classification (CPC):
**A61B 34/71; A61B 34/37; A61B 34/77;**
A61B 2034/715

(86) International application number:
**PCT/US2022/019703**

(87) International publication number:
**WO 2022/192509 (15.09.2022 Gazette 2022/37)**

(54) **SURGICAL ROBOTIC SYSTEM FOR REALIGNMENT OF WRISTED INSTRUMENTS**

CHIRURGISCHES ROBOTERSYSTEM ZUR NEUAUSRICHTUNG VON HANDGEFÜHRTEN
INSTRUMENTEN

SYSTÈME ROBOTIQUE CHIRURGICAL POUR LE RÉALIGNEMENT DES INSTRUMENTS DE
POIGNET

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.03.2021 US 202163159559 P**

(43) Date of publication of application:
**17.01.2024 Bulletin 2024/03**

(73) Proprietor: **Covidien LP**
**Mansfield, MA 02048 (US)**

(72) Inventors:
• **PEINE, William**
 **Boston, Massachusetts 02210 (US)**
• **YILMAZ, Burak**
 **Boston, Massachusetts 02210 (US)**

(74) Representative: **Maschio & Soames IP Ltd**
**30 Carlton Crescent**
**Southampton SO15 2EW (GB)**

(56) References cited:
**WO-A1-2016/201207  WO-A1-2020/118149**
**US-A1- 2006 106 493  US-A1- 2020 405 405**
**US-A1- 2021 045 826  US-A1- 2021 059 777**
**US-B2- 10 595 946**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

CROSS-REFERENCE TO RELATED APPLICATION

The present application claims priority to U.S. Patent Application No. 63/159,559, filed on March 11, 2021.

BACKGROUND

*Technical Field*

The present disclosure generally relates to a surgical robotic system having one or more modular arm carts each of which supports a robotic arm, and a surgical console for controlling the carts and their respective arms. More particularly, the present disclosure is directed to a system and method for controlling an instrument through an instrument drive unit of the robotic arm to realign the instrument and/or the jaws in response to detection of misalignment.

*Background of Related Art*

Surgical robotic systems are currently being used in minimally invasive medical procedures. Some surgical robotic systems include a surgical console controlling a surgical robotic arm and a surgical instrument having an end effector (e.g., forceps or grasping instrument) coupled to and actuated by the robotic arm. In operation, the robotic arm is moved to a position over a patient and then guides the surgical instrument into a small incision via a surgical port or a natural orifice of a patient to position the end effector at a work site within the patient's body.
Due to remote operation of surgical robotic instruments, actuation of the instrument needs to accurately reflect the movement commands input remotely at a surgical console. Thus, there is a need to provide for precise operation of the surgical robotic instruments. US patent application US2021/045826 describes roll joint engagment for a sugical tool and PCT application WO 2020/118149 describes a method of controlling cable driven end effectors.

SUMMARY

According to a first aspect is provided a surgical robotic system comprising: a surgical console including a handle controller; a robotic arm including an instrument having: a plurality of cables that are movable longitudinally; and an end effector movable by the cables, the end effector having a pair of opposing jaws; and a processor configured to: determine an amount of orientation an instrument tool center point, and adjust the instrument based on the amount of orientation misalignment of he instrument tool center point;
wherein the processor is further configured to calculate a motion axis based on a difference between a current instrument desired orientation and a previous instrument desired orientation, the current instrument desired orientation being based on an orientation of the handle controller; wherein the processor is further configured to calculate a user delta orientation input based on a difference between a current orientation of the handle controller and a previous orientation of the handle controller; wherein the processor is further configured to calculate an unadjusted instrument commanded orientation by adding the user delta orientation input to a previous instrument commanded orientation; wherein the processor is further configured to calculate a misalignment axis, which is a difference between the current instrument desired orientation and the unadjusted instrument commanded orientation; wherein the processor is further configured to calculate an adjustment value based on an angle between the motion axis and the misalignment axis, characterized wherein the processor is further configured to calculate the adjustment value using a first negative punishing scale in response to a cosine of the angle being positive; , wherein the processor is further configured to calculate the adjustment value using a second negative punishing scale in response to a cosine of the angle being negative, the second negative punishing scale being larger than the first negative punishing scale in absolute value.

BRIEF DESCRIPTION OF THE DRAWINGS

Various embodiments of the present disclosure are described herein with reference to the drawings wherein:

FIG. 1 is a schematic illustration of a surgical robotic system including a control tower, a console, and one or more surgical robotic arms according to an embodiment of the present disclosure;

FIG. 2 is a perspective view of a surgical robotic arm of the surgical robotic system of FIG. 1 according to an embodiment of the present disclosure;

FIG. 3 is a perspective view of a setup arm with the surgical robotic arm of the surgical robotic system of FIG. 1 according to an embodiment of the present disclosure;

FIG. 4 is a schematic diagram of a computer architecture of the surgical robotic system of FIG. 1 according to an embodiment of the present disclosure;

FIG. 5 is a perspective view of an instrument drive unit and a surgical instrument according to an embodiment of the present disclosure;

FIG. 6 is a perspective view, with parts separated, of the instrument drive unit and the surgical instrument shown in FIG. 5 according to an embodiment of the present disclosure;

FIG. 7 is a rear perspective view of the surgical instrument for use with the robotic surgical assembly of FIGS. 5 and 6;

FIG. 8 is a perspective view of drive assemblies of the surgical instrument of FIG. 7;

FIG. 9A is a cross-sectional view of the surgical instrument, as taken through 9A-9A of FIG. 7;

FIG. 9B is a cross-sectional view of the surgical instrument, as taken through 9B-9B of FIG. 7;

FIG. 10A is a top, perspective view of an end effector, according to an embodiment of the present disclosure, for use in the surgical robotic system of FIG. 1;

FIG. 10B is a side view of a stapler for use the surgical robotic system of FIG. 1 according to an embodiment of the present disclosure;

FIG. 10C is a side view of an electrocautery shears for use the surgical robotic system of FIG. 1 according to an embodiment of the present disclosure;

FIG. 10D is a side view of an electrocautery blade for use the surgical robotic system of FIG. 1 according to an embodiment of the present disclosure;

FIG. 10E is a side view of an electrosurgical vessel sealer for use the surgical robotic system of FIG. 1 according to an embodiment of the present disclosure;

FIG. 11 is a flow control diagram of components controlling position of the end effector of FIG. 10A according to an embodiment of the present disclosure; and

FIGS. 12A and 12B are flow charts of methods for alignment of the surgical instrument according to an embodiment of the present disclosure.

DETAILED DESCRIPTION

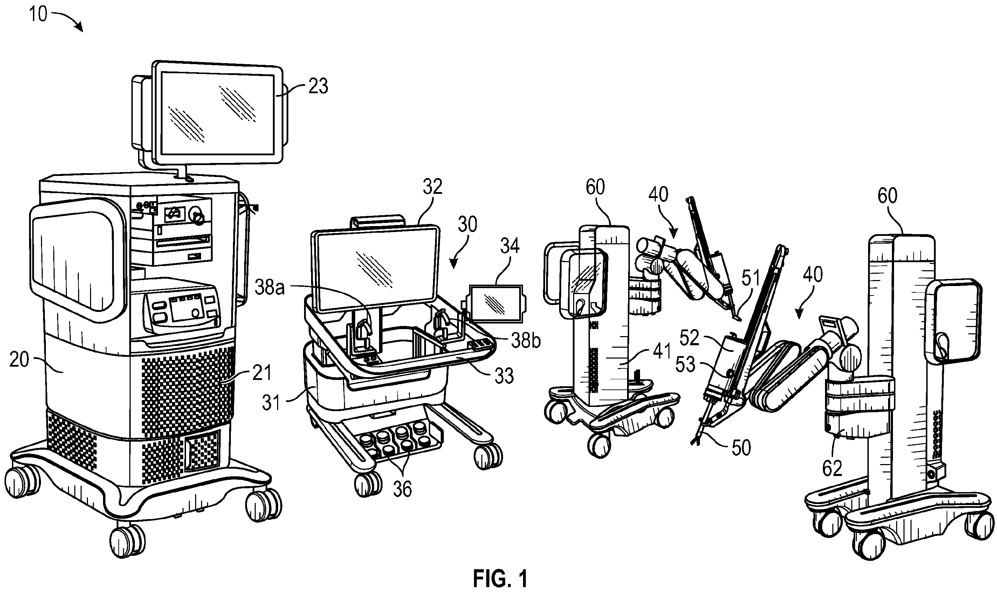

Embodiments of the presently disclosed surgical robotic system are described in detail with reference to the drawings, in which like reference numerals designate identical or corresponding elements in each of the several views. As used herein the term "distal" refers to the portion of the surgical robotic system and/or the surgical instrument coupled thereto that is closer to the patient, while the term "proximal" refers to the portion that is farther from the patient.
The term "application" may include a computer program designed to perform functions, tasks, or activities for the benefit of a user. Application may refer to, for example, software running locally or remotely, as a standalone program or in a web browser, or other software which would be understood by one skilled in the art to be an application. An application may run on a controller, or on a user device, including, for example, a mobile device, a personal computer, or a server system.
As will be described in detail below, the present disclosure is directed to a surgical robotic system, which includes a surgical console, a control tower, and one or more movable carts having a surgical robotic arm coupled to a setup arm. The surgical console receives user input through one or more interface devices, which are interpreted by the control tower as movement commands for moving the surgical robotic arm. The surgical robotic arm includes a controller, which is configured to process the movement command and to generate a torque command for activating one or more actuators of the robotic arm, which would, in turn, move the robotic arm in response to the movement command.
With reference to FIG. 1, a surgical robotic system 10 includes a control tower 20, which is connected to all of the components of the surgical robotic system 10 including a surgical console 30 and one or more robotic arms 40. Each of the robotic arms 40 includes a surgical instrument 50 removably coupled thereto. Each of the robotic arms 40 is also coupled to a movable cart 60.

The surgical instrument 50 is configured for use during minimally invasive surgical procedures. In embodiments, the surgical instrument 50 may be configured for open surgical procedures. In embodiments, the surgical instrument 50 may be an endoscope, such as an endoscopic camera 51, configured to provide a video feed for the user. In further embodiments, the surgical instrument 50 may be an electrosurgical forceps configured to seal tissue by compressing tissue between jaw members and applying electrosurgical current thereto. In yet further embodiments, the surgical instrument 50 may be a surgical stapler including a pair of jaws configured to grasp and clamp tissue while deploying a plurality of tissue fasteners, e.g., staples, and cutting stapled tissue.

One of the robotic arms 40 may include a camera 51 configured to capture video of the surgical site. The surgical console 30 includes a first display 32, which displays a video feed of the surgical site provided by camera 51 of the surgical instrument 50 disposed on the robotic arms 40, and a second display 34, which displays a user interface for controlling the surgical robotic system 10. The first and second displays 32 and 34 are touchscreens allowing for displaying various graphical user inputs.

The surgical console 30 also includes a plurality of user interface devices, such as foot pedals 36 and a pair of handle controllers 38a and 38b which are used by a user to remotely control robotic arms 40. The surgical console further includes an armrest 33 used to support clinician's arms while operating the handle controllers 38a and 38b.

The control tower 20 includes a display 23, which may be a touchscreen, and outputs on the graphical user interfaces (GUIs). The control tower 20 also acts as an interface between the surgical console 30 and one or more robotic arms 40. In particular, the control tower 20 is configured to control the robotic arms 40, such as to move the robotic arms 40 and the corresponding surgical instrument 50, based on a set of programmable instructions and/or input commands from the surgical console 30, in such a way that robotic arms 40 and the surgical instrument 50 execute a desired movement sequence in response to input from the foot pedals 36 and the handle controllers 38a and 38b.

Each of the control tower 20, the surgical console 30, and the robotic arm 40 includes a respective computer 21, 31, 41. The computers 21, 31, 41 are interconnected to each other using any suitable communication network based on wired or wireless communication protocols. The term "network," whether plural or singular, as used herein, denotes a data network, including, but not limited to, the Internet, Intranet, a wide area network, or a local area networks, and without limitation as to the full scope of the definition of communication networks as encompassed by the present disclosure. Suitable protocols include, but are not limited to, transmission control protocol/internet protocol (TCP/IP), datagram protocol/internet protocol (UDP/IP), and/or datagram congestion control protocol (DCCP). Wireless communication may be achieved via one or more wireless configurations, e.g., radio frequency, optical, Wi-Fi, Bluetooth (an open wireless protocol for exchanging data over short distances, using short length radio waves, from fixed and mobile devices, creating personal area networks (PANs), ZigBee® (a specification for a suite of high level communication protocols using small, low-power digital radios based on the IEEE 122.15.4-2003 standard for wireless personal area networks (WPANs)).

The computers 21, 31, 41 may include any suitable processor (not shown) operably connected to a memory (not shown), which may include one or more of volatile, non-volatile, magnetic, optical, or electrical media, such as read-only memory (ROM), random access memory (RAM), electrically-erasable programmable ROM (EEPROM), non-volatile RAM (NVRAM), or flash memory. The processor may be any suitable processor (e.g., control circuit) adapted to perform the operations, calculations, and/or set of instructions described in the present disclosure including, but not limited to, a hardware processor, a field programmable gate array (FPGA), a digital signal processor (DSP), a central processing unit (CPU), a microprocessor, and combinations thereof. Those skilled in the art will appreciate that the processor may be substituted for by using any logic processor (e.g., control circuit) adapted to execute algorithms, calculations, and/or set of instructions described herein.

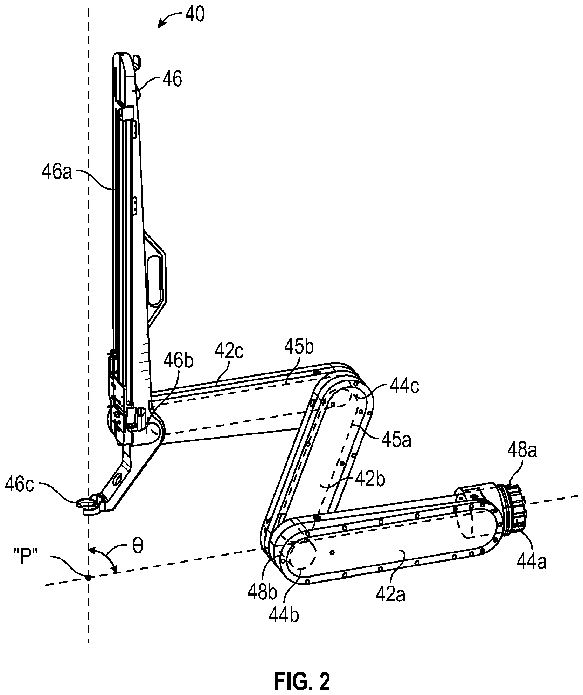

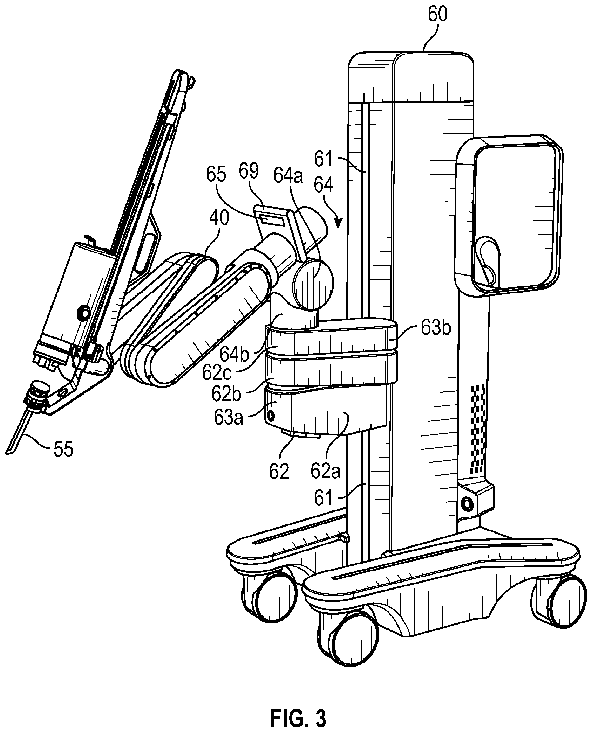

With reference to FIG. 2, each of the robotic arms 40 may include a plurality of links 42a, 42b, 42c, which are interconnected at joints 44a, 44b, 44c, respectively. The joint 44a is configured to secure the robotic arm 40 to the movable cart 60 and defines a first longitudinal axis. With reference to FIG. 3, the movable cart 60 includes a lift 61 and a setup arm 62, which provides a base for mounting of the robotic arm 40. The lift 61 allows for vertical movement of the setup arm 62. The movable cart 60 also includes a display 69 for displaying information pertaining to the robotic arm 40.

The setup arm 62 includes a first link 62a, a second link 62b, and a third link 62c, which provide for lateral maneuverability of the robotic arm 40. The links 62a, 62b, 62c are interconnected at joints 63a and 63b, each of which may include an actuator (not shown) for rotating the links 62b and 62b relative to each other and the link 62c. In particular, the links 62a, 62b, 62c are movable in their corresponding lateral planes that are parallel to each other, thereby allowing for extension of the robotic arm 40 relative to the patient (e.g., surgical table). In embodiments, the robotic arm 40 may be coupled to the surgical table (not shown). The setup arm 62 includes controls 65 for adjusting movement of the links 62a, 62b, 62c as well as the lift 61.

The third link 62c includes a rotatable base 64 having two degrees of freedom. In particular, the rotatable base 64 includes a first actuator 64a and a second actuator 64b. The first actuator 64a is rotatable about a first stationary arm axis which is perpendicular to a plane defined by the third link 62c and the second actuator 64b is rotatable about a second stationary arm axis which is transverse to the first stationary arm axis. The first and second actuators 64a and 64b allow for full three-dimensional orientation of the robotic arm 40.

The actuator 48b of the joint 44b is coupled to the joint 44c via the belt 45a, and the joint 44c is in turn coupled to the joint 46c via the belt 45b. Joint 44c may include a transfer case coupling the belts 45a and 45b, such that the actuator 48b is configured to rotate each of the links 42b, 42c and the holder 46 relative to each other. More specifically, links 42b, 42c, and the holder 46 are passively coupled to the actuator 48b which enforces rotation about a pivot point "P" which lies at an intersection of the first axis defined by the link 42a and the second axis defined by the holder 46. Thus, the actuator 48b controls the angle $\theta$ between the first and second axes allowing for orientation of the surgical instrument 50. Due to the interlinking of the links 42a, 42b, 42c, and the holder 46 via the belts 45a and 45b, the angles between the links 42a, 42b, 42c, and the holder 46 are also adjusted in order to achieve the desired angle $\theta$. In embodiments, some or all of the joints 44a, 44b, 44c may include an actuator to obviate the need for mechanical linkages.

The joints 44a and 44b include an actuator 48a and 48b configured to drive the joints 44a, 44b, 44c relative to each other through a series of belts 45a and 45b or other mechanical linkages such as a drive rod, a cable, or a lever and the like. In particular, the actuator 48a is configured to rotate the robotic arm 40 about a longitudinal axis defined by the link 42a.

With reference to FIG. 2, the robotic arm 40 also includes a holder 46 defining a second longitudinal axis and configured to receive an instrument drive unit (IDU) 52 (FIG. 1). The IDU 52 is configured to couple to an actuation mechanism of the surgical instrument 50 and the camera 51 and is configured to move (e.g., rotate) and actuate the instrument 50 and/or the camera 51. IDU 52 transfers actuation forces from its actuators to the surgical instrument 50 to actuate components (e.g., end effector) of the surgical instrument 50. The holder 46 includes a sliding mechanism 46a, which is configured to move the IDU 52 along the second longitudinal axis defined by the holder 46. The holder 46 also includes a joint 46b, which rotates the holder 46 relative to the link 42c. During endoscopic procedures, the instrument 50 may be inserted through an endoscopic port 55 (FIG. 3) held by the holder 46.

The robotic arm 40 also includes a plurality of manual override buttons 53 (FIG. 1) disposed on the IDU 52 and the setup arm 62, which may be used in a manual mode. The user may press one or more of the buttons 53 to move the component associated with the button 53.

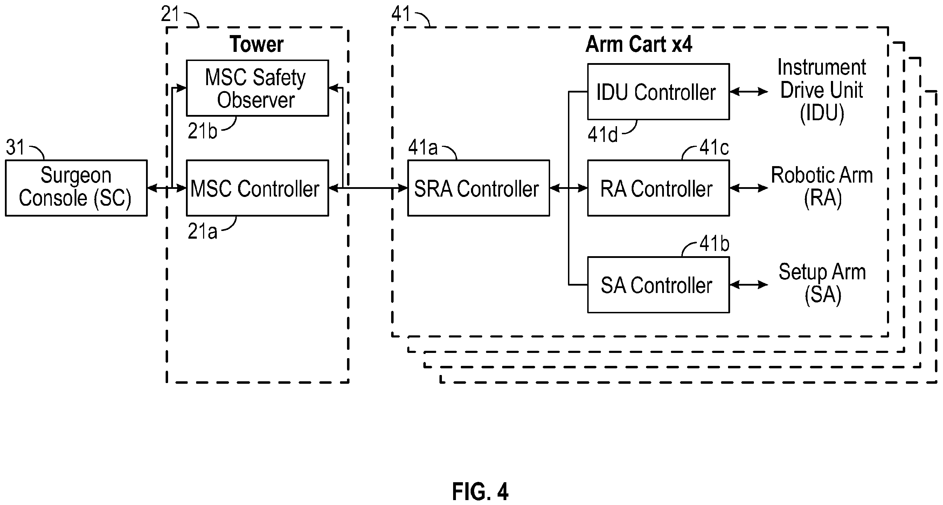

With reference to FIG. 4, each of the computers 21, 31, 41 of the surgical robotic system 10 may include a plurality of controllers, which may be embodied in hardware and/or software. The computer 21 of the control tower 20 includes a controller 21a and safety observer 21b. The controller 21a receives data from the computer 31 of the surgical console 30 about the current position and/or orientation of the handle controllers 38a and 38b and the state of the foot pedals 36 and other buttons. The controller 21a processes these input positions to determine desired drive commands for each joint of the robotic arm 40 and/or the IDU 52 and communicates these to the computer 41 of the robotic arm 40. The controller 21a also receives the actual joint angles measured by encoders of the actuators 48a and 48b and uses this information to determine force feedback commands that are transmitted back to the computer 31 of the surgical console 30 to provide haptic feedback through the handle controllers 38a and 38b. The safety observer 21b performs validity checks on the data going into and out of the controller 21a and notifies a system fault handler if errors in the data transmission are detected to place the computer 21 and/or the surgical robotic system 10 into a safe state.

The computer 41 includes a plurality of controllers, namely, a main cart controller 41a, a setup arm controller 41b, a robotic arm controller 41c, and an instrument drive unit (IDU) controller 41d. The main cart controller 41a receives and processes joint commands from the controller 21a of the computer 21 and communicates them to the setup arm controller 41b, the robotic arm controller 41c, and the IDU controller 41d. The main cart controller 41a also manages instrument exchanges and the overall state of the movable cart 60, the robotic arm 40, and the IDU 52. The main cart controller 41a also communicates actual joint angles back to the controller 21a.

The setup arm controller 41b controls each of joints 63a and 63b, and the rotatable base 64 of the setup arm 62 and calculates desired motor movement commands (e.g., motor torque) for the pitch axis and controls the brakes. The robotic arm controller 41c controls each joint 44a and 44b of the robotic arm 40 and calculates desired motor torques required for gravity compensation, friction compensation, and closed loop position control of the robotic arm 40. The robotic arm controller 41c calculates a movement command based on the calculated torque. The calculated motor commands are then communicated to one or more of the actuators 48a and 48b in the robotic arm 40. The actual joint positions are then transmitted by the actuators 48a and 48b back to the robotic arm controller 41c.

The IDU controller 41d receives desired joint angles for the surgical instrument 50, such as wrist and jaw angles, and computes desired currents for the motors in the IDU 52. The IDU controller 41d calculates actual angles based on the motor positions and transmits the actual angles back to the main cart controller 41a.

The robotic arm 40 is controlled in response to a pose of the handle controller controlling the robotic arm 40, e.g., the handle controller 38a, which is transformed into a desired pose of the robotic arm 40 through a hand eye transform function executed by the controller 21a. The hand eye function, as well as other functions described herein, is/are embodied in software executable by the controller 21a or any other suitable controller described herein. The pose of one of the handle controller 38a may be embodied as a coordinate position and role-pitch-yaw ("RPY") orientation relative to a coordinate reference frame, which is fixed to the surgical console 30. The desired pose of the instrument 50 is relative to a fixed frame on the robotic arm 40. The pose of the handle controller 38a is then scaled by a scaling function executed by the controller 21a. In embodiments, the coordinate position is scaled down and the orientation is scaled up by the scaling function. In addition, the controller 21a also executes a clutching function, which disengages the handle controller 38a from the robotic arm 40. In particular, the controller 21a stops transmitting movement commands from the handle controller 38a to the robotic arm 40 if certain movement limits or other thresholds are exceeded and in essence acts like a virtual clutch mechanism, e.g., limits mechanical input from effecting mechanical output.

The desired pose of the robotic arm 40 is based on the pose of the handle controller 38a and is then passed by an inverse kinematics function executed by the controller 21a. The inverse kinematics function calculates angles for the joints 44a, 44b, 44c of the robotic arm 40 that achieve the scaled and adjusted pose input by the handle controller 38a. The calculated angles are then passed to the robotic arm controller 41c, which includes a joint axis controller having a proportional-derivative (PD) controller, the friction estimator module, the gravity compensator module, and a two-sided saturation block, which is configured to limit the commanded torque of the motors of the joints 44a, 44b, 44c.

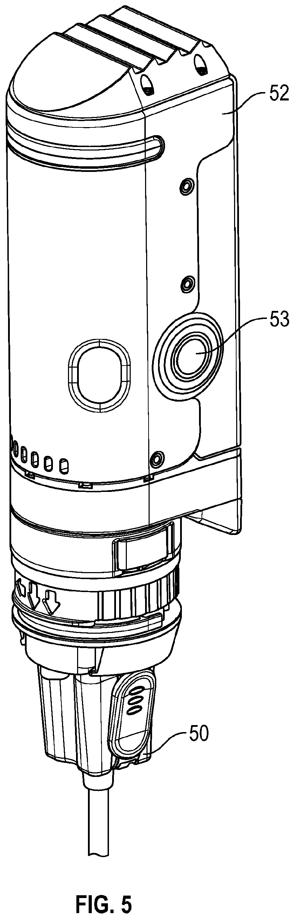

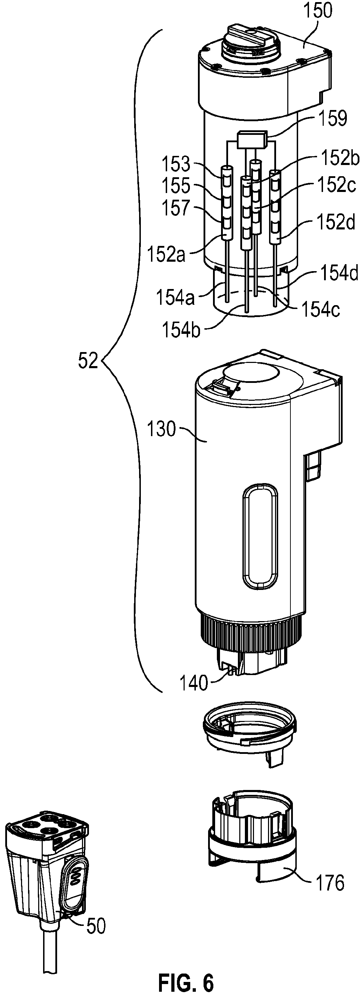

With reference to FIGS. 5 and 6, the IDU 52 is shown in more detail and is configured to transfer power and actuation forces from its motors 152a, 152b, 152c, 152d to the instrument 50 to drive movement of components of the instrument 50, such as articulation, rotation, pitch, yaw, clamping, cutting, etc. The IDU 52 may also be configured for the activation or firing of an electrosurgical energy-based instrument or the like (e.g., cable drives, pulleys, friction wheels, rack and pinion arrangements, etc.).

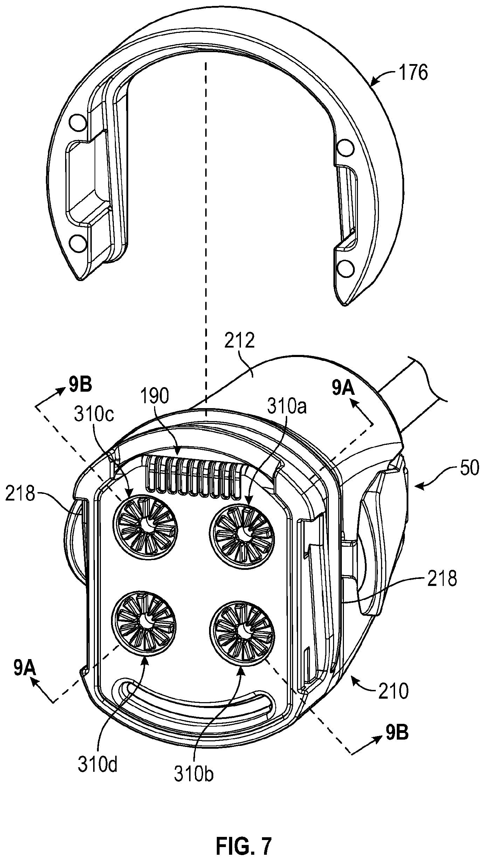

The IDU 52 includes a motor pack 150 and a sterile barrier housing 130. Motor pack 150 includes motors 152a, 152b, 152c, 152d for controlling various operations of the instrument 50. The instrument 50 is removably coupleable to IDU 52. As the motors 152a, 152b, 152c, 152d of the motor pack 150 are actuated, rotation of the drive transfer shafts 154a, 154b, 154c, 154d of the motors 152a, 152b, 152c, 152d, respectively, is transferred to the respective proximal couplers 310a, 310b, 310c, 310d of the drive assemblies 300a, 300b, 300c, 300d (FIG. 7) of the instrument 50.

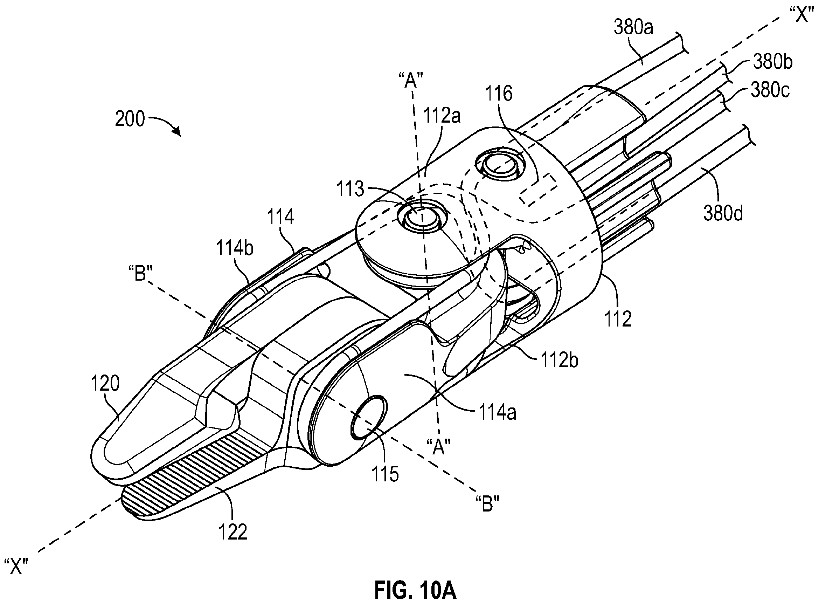

The instrument 50 may have an end effector, which shown as a needle driver 200 (FIG. 10A) secured to a distal end thereof. The instrument 50 is configured to transfer rotational forces/movement supplied by the IDU 52 (e.g., via the motors 152a, 152b, 152c, 152d of the motor pack 150) into longitudinal movement or translation of the cables 380a, 380b, 380c, 380d to effect various functions of the needle driver 200.

With reference to FIGS. 7-9B, the instrument 50 includes a housing assembly 210 with a housing 212 defining at least one cavity or bore 212a, 212b, 212c, 212d therein which is configured to receive a respective drive assembly 300a, 300b, 300c, 300d therein. In accordance with the present disclosure, each bore 212a, 212b, 212c, 212d of the housing 212 is configured to operatively support a respective drive assembly 300a, 300b, 300c, 300d therein.

Each bore 212a, 212b, 212c, 212d of the housing 212 defines a respective longitudinally extending groove or channel 213a, 213b, 213c, 213d therein. Each channel 213a, 213b, 213c, 213d is configured to slidingly accept a rail or tab 353a, 353b, 353c, 353d extending radially from a respective drive nut 350a, 350b, 350c, 350d of a respective drive assembly 300a, 300b, 300c, 300d.

When the instrument 50 is connected to the IDU 52, the proximal couplers 310a, 310b, 310c, 310d of the drive assemblies 300a, 300b, 300c, 300d of the instrument 50 come into registration with and are connected to respective drive transfer shafts 154a, 154b, 154c, 154d within the IDU 52 (FIGS. 5 and 6) to couple the respective drive assemblies 300a, 300b, 300c, 300d to respective motors 152a, 152b, 152c, 152d of the IDU 52.

The housing 212 of the housing assembly 210 of the instrument 50 also supports an electrical connector 190 (FIG. 7) configured for selective connection to the plug 140 of the IDU 52 (FIGS. 5 and 6) of the IDU 52. The instrument 50 may include electronics, including, and not limited to, a memory (for storing identification information, usage information, and the like), wired or wireless communication circuitry for receiving and transmitting data or information. The IDU 52 may be configured to permit passage or routing of a dedicated electrocautery cable or the like for use and connection to an electrosurgical based electromechanical surgical instrument (e.g., for ablation, coagulation, sealing, etc.). The electrical connector 190 may include and is not limited to conductive connectors, magnetic connectors, resistive connectors, capacitive connectors, Hall sensors, reed switches or the like. The instrument 50 may be secured to the IDU 52 via a coupler 176 configured to engage a guide 218.

With continued reference to FIGS. 7-9B, the housing assembly 210 of the instrument 50 houses a plurality of drive assemblies, shown as drive assemblies 300a, 300b, 300c, 300d. In the illustrated embodiment, the instrument 50 includes three drive assemblies 300a, 300b, 300c, 300d; however, the instrument 50 may include more (e.g., four, five, or six) or fewer (e.g., two) drive assemblies without departing from the scope of the present disclosure.

Each drive assembly 300a, 300b, 300c, 300d includes a respective proximal coupler 310a, 310b, 310c, 310d, a proximal bearing 320a, 320b, 320c, 320d, a drive screw 340a, 340b, 340c, 340d, a drive nut 350a, 350b, 350c, 350d, a biasing element 370a, 370b, 370c, 370d, and a cable 380a, 380b, 380c, 380d. The proximal coupler 310a, 310b, 310c, 310d of each drive assembly 300a, 300b, 300c, 300d is configured to meshingly engage with respective drive transfer shafts 154a, 154b, 154c, 154d coupled to respective motors of the IDU 52. In operation, rotation of the drive transfer shafts 154a, 154b, 154c, 154d of the motors 152a, 152b, 152c, 152d results in corresponding rotation of respective proximal coupler 310a, 310b, 310c, 310d of respective drive assembly 300a, 300b, 300c, 300d.

The proximal coupler 310a, 310b, 310c, 310d of each drive assembly 300a, 300b, 300c, 300d is keyed to or otherwise non-rotatably connected to a proximal end of a respective drive screw 340a, 340b, 340c, 340d. Accordingly, rotation of the proximal coupler 310a, 310b, 310c, 310d results in a corresponding rotation of a respective drive screw 340a, 340b, 340c, 340d.

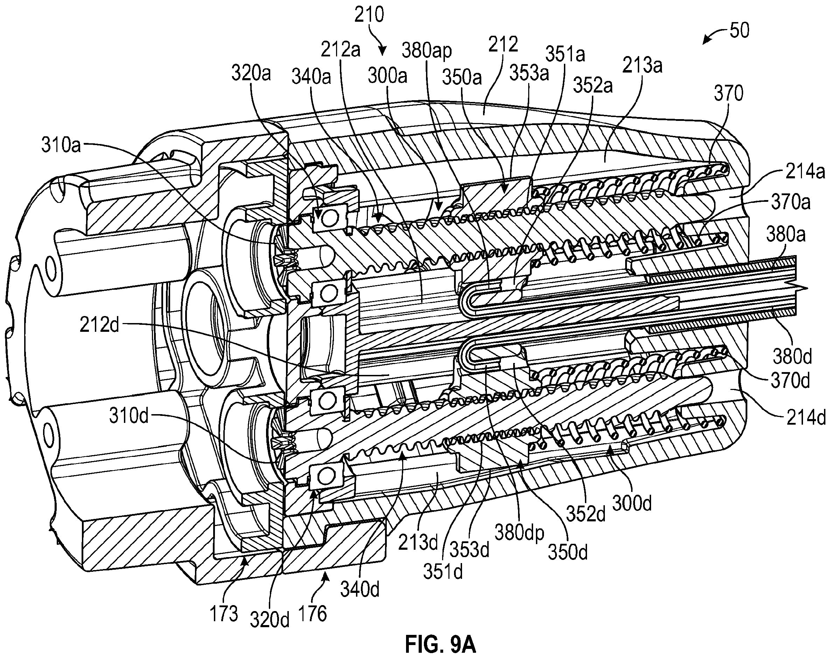

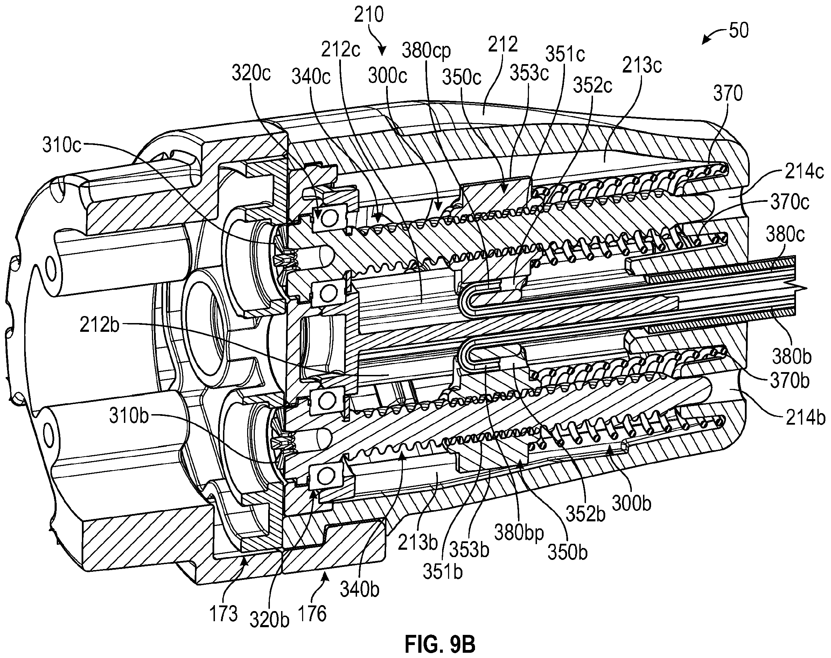

Each proximal bearing 320a, 320b, 320c, 320d is disposed about a proximal portion of a respective drive screw 340a, 340b, 340c, 340d adjacent a proximal end of the housing 212 of the housing assembly 210. A distal end or tip of each drive screw 340a, 340b, 340c, 340d may be rotatably disposed or supported in a respective recess 214a, 214b, 214c, 214d defined in a distal end of the housing 212 (see FIGS. 9A-B).

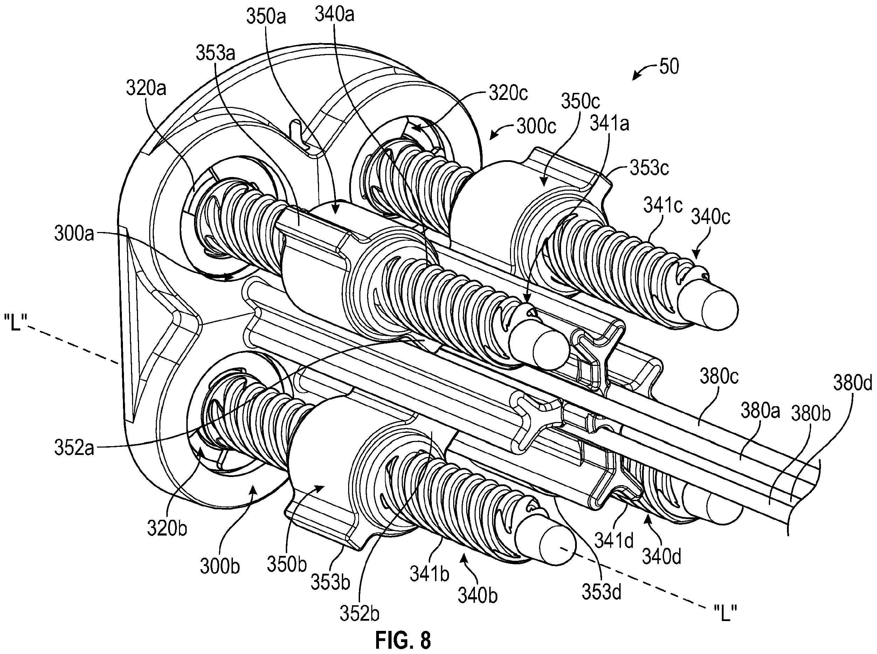

Each of the drive screws 340a, 340b, 340c, 340d includes a threaded body or shaft portion 341a, 341b, 341c, 341d and defines a longitudinal axis "L-L" extending through a radial center thereof (see FIG. 8). In use, rotation of the proximal coupler 310a, 310b, 310c, 310d as described above, results in rotation of a respective drive screw 340a, 340b, 340c, 340d about longitudinal axis "L-L", in a corresponding direction and rate of rotation.

Each of the drive nuts 350a, 350b, 350c, 350d includes a threaded aperture 351a, 351b, 351c, 315d extending longitudinally therethrough, which is configured to mechanically engage the threaded shaft portion 341a, 341b, 341c, 341d of a respective drive screw 340a, 340b, 340c, 340d. Each drive nut 350a, 350b, 350c, 350d is configured to be positioned on a respective drive screw 340a, 340b, 340c, 340d in a manner such that rotation of the drive screw 340a, 340b, 340c, 340d causes longitudinal movement or translation of the respective drive nut 350a, 350b, 350c, 350d. Moreover, rotation of the proximal coupler 310a, 310b, 310c, 310d in a first direction (e.g., clockwise) causes the respective drive nut 350a, 350b, 350c, 350d to move in a first longitudinal direction (e.g., proximally) along the respective drive screw 340a, 340b, 340c, 340d and rotation of the proximal coupler 310a, 310b, 310c, 310d in a second direction (e.g., counter-clockwise) causes the respective drive nut 350a, 350b, 350c, 350d to move in a second longitudinal direction (e.g., distally) with respect to the respective drive screw 340a, 340b, 340c, 340d.

Each drive nut 350a, 350b, 350c, 350d includes a retention pocket formed in an engagement tab 352a, 352b, 352c, 352d formed therein that is disposed adjacent the threaded aperture 351a, 351b, 351c, 351d thereof. Each retention pocket is configured to retain a proximal end portion 380ap, 380bp, 380cp, 380dp of a respective cable 380a, 380b, 380c, 380d, as discussed in further detail below.

Each drive nut 350a, 350c, 350c, 350d also includes a tab 353a, 353b, 353c, 353d extending radially from and longitudinally along an outer surface thereof. The tab 353a, 353b, 353c, 353d of each drive nut 350a, 350b, 350c, 350d is configured to be slidably disposed in a respective longitudinally extending channel 213a, 213b, 213c, 213d formed in the bores 212a, 212b, 212c, 212d of the housing 212. The tab 353a, 353b, 353c, 353d of each drive nut 350a, 350b, 350c, 350d cooperates with a respective channel 213a, 213b, 213c, 213d of the bore 212a, 212b, 212c, 212d of the housing 212 to inhibit or prevent each drive nut 350a, 350b, 350c, 350d from rotating about longitudinal axis "L-L" as each drive screw 340a, 340b, 340c, 340d is rotated.

The engagement tabs 352a, 352b, 352c, 352d of each of the drive nuts 350a, 350b, 350c, 350d includes is disposed adjacent a radially inward surface thereof, which is configured to mechanically engage or retain a proximal end portion 380ap, 380bp, 380cp, 380dp of a respective cable 380a, 380b, 380c, 380d. In operation, as the drive nuts 350a, 350b, 350c, 350d are axially displaced along the drive screw 340a, 340b, 340c, 340d, the drive nuts 350a, 350b, 350c, 350d transmit concomitant axial translation to the cable 380a, 380b, 380c, 380d.

The biasing elements 370a, 370b, 370c, 370d, which may be compression springs, are configured to radially surround a respective distal portion of the threaded shaft portion 341a, 341b, 341c, 341d of each drive screw 340a, 340b, 340c, 340d. Each biasing element 370a, 370b, 370c, 370d is interposed between a respective drive nut 350a, 350b, 350c, 350d and a distal surface of the housing 212 of the housing assembly 210.

Each cable 380a, 380b, 380c, 380d extends distally from a respective drive nut 350a, 350b, 350c, through a respective central bore or channel 212a, 212b, 212c, 212d of the housing 212 of the housing assembly 210 and is configured to mechanically engage a portion of a surgical instrument, e.g., a portion or component of needle driver 200, of the instrument 50.

In operation, longitudinal translation of at least one cable 380a, 380b, 380c, 380d is configured to move and/or actuate the needle driver 200 of the instrument 50 in a specific manner. With reference to FIG. 10A, the needle driver 200 includes a proximal portion 112 having a first pin 113 and a distal portion 114. The distal portion 114 is pivotable about the pin 113, which defines an axis "A-A." The cables 380a, 380b, 380c, 380d are routed through proximal and distal portions 112 and 114 around their respective pulleys 112a, 112b, 114a, 114b, which are integrally formed as arms of the proximal and distal portions 112 and 114. In embodiments, the needle driver 200, namely, the distal portion 114 and the jaws 120 and 122, may be articulated about the axis "A-A" to control a yaw angle of the end effector with respect to a longitudinal axis "X-X". The distal portion 114 includes a second pin 115 with a pair of jaws 120 and 122 pivotably coupled to the second pin 115. The jaws 120 and 122 configured to pivot about an axis "B-B" defined by the second pin 115 allowing for controlling a pitch angle of the jaws 120 and 122 as well as opening and closing the jaws 120 and 122. The yaw, pitch, and jaw angles are controlled by adjusting the tension and/or length and direction (e.g., proximal or distal) of the cables 380a, 380b, 380c, 380d. The needle driver 200 also includes a cable displacement sensor 116 configured to measure position of the cables 380a, 380b, 380c, 380d. Thus, the needle driver 200 has three degrees of freedom, yaw, pitch and jaw angle between jaws 120 and 122. In embodiments, the needle driver 200 may have a plurality of cable displacement sensors 116, one for each of the cables 380a, 380b, 380c, 380d. The cable displacement sensor 116 is stationary and may operate by measuring a particular movable point on the cable 380a, 380b, 380c, 380d marked by a marker (e.g., magnet, dot, etc.). The cable displacement sensor 116 may be an optical encoder, a Hall Effect sensor, or any other suitable displacement sensor.

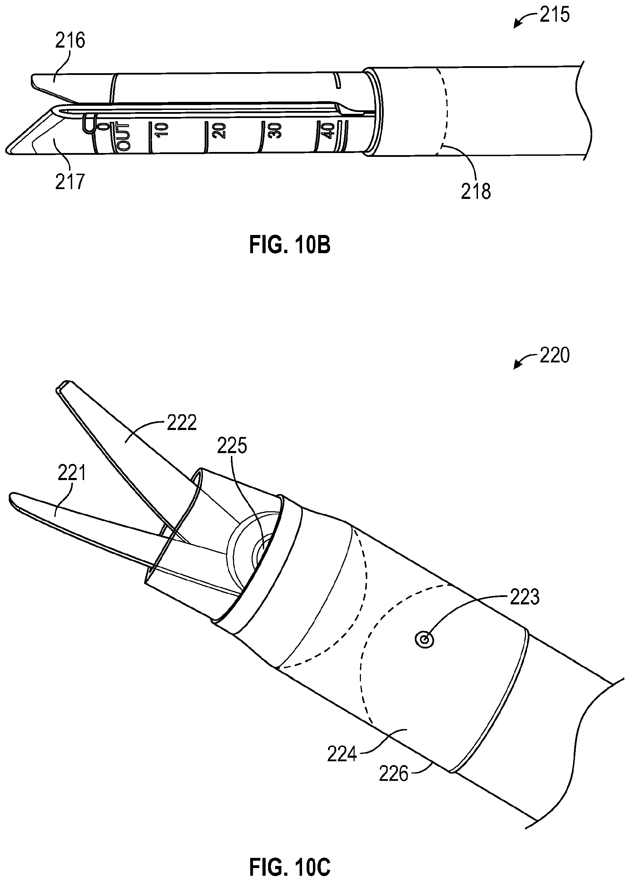

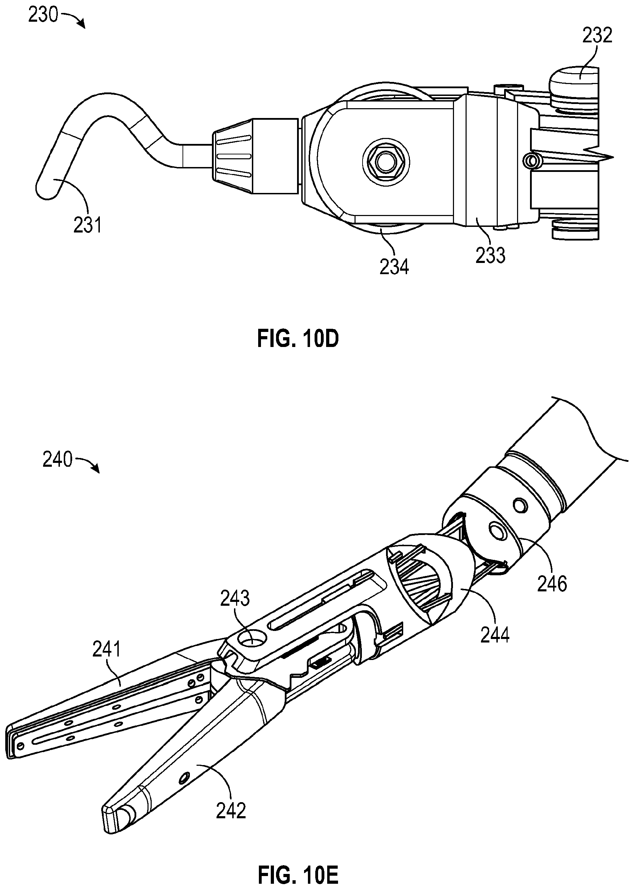

In addition to the needle driver 200 of FIG. 10A, the system 10 may use other types of end effectors as shown in FIGS. 10B-E, such as a stapler 215 (FIG. 10B), shears 220 (FIG. 10C), electrocautery blade 230 (FIG. 10E), and a vessel sealer 240 (FIG. 10E). Reference is made below to the proximal and distal portions 112 and 114 as well as to pivot pins 113 and 115 as well their respective pivot axes "A-A" and "B-B" using different numerals to avoid confusion.

The stapler 215 of FIG. 10B is a jaw-type instrument having a first jaw 216, which is an anvil, and a second jaw 217, which includes a staple cartridge housing a plurality of staples ejectable against the first jaw 216. The stapler 215 may be pivotally coupled to a longitudinal shaft 215 via a pin 218, such that the stapler 215 may be articulated about the axis "A-A."

The shears 220 of FIG. 10C may be monopolar curved shears or any other scissor-like cutting end effector and includes a first blade 221 and a second blade 222 that are pivotable relative to each other about a second pin 225 and the axis "B-B". The first and second blades 221 and 222 are energizable by an electrosurgical generator (not shown) operating in a monopolar mode, such that the shears 220 simultaneously cuts and cauterizes tissue. The shears 220 may also include an insulating cover 226 disposed over a proximal portion of the shears 220 to prevent incidental contact with the tissue. The shears 220 may also include a proximal portion 224 and a first pin 223, such that the shears 220 may be articulated about the axis "A-A."

The electrocautery blade 230 of FIG. 10D includes a cutting element 231, which may be a hook or any suitable monopolar electrode. The electrocautery blade 230 also includes one or more pivotable distal and proximal portions 232 and 234 and may be pivotable about one or more of the pivot axes "A-A" and "B-B." The electrocautery blade 230 is also energized by the electrosurgical generator operating in a monopolar mode. In addition, the electrocautery blade 230 also includes an insulating cover 233 disposed over the proximal and distal portions 232 and 234.

The vessel sealer 240 of FIG. 10E includes a pair of jaws 241 and 242 each having an electroconductive surface. The jaws 241 and 242 are configured to pivot relative to each other about a pin 243. In addition, the vessel sealer 240 may include one or more pivotable distal and proximal portions 244 and 246 and may be pivotable about one or more of the pivot axes "A-A" and "B-B." The vessel sealer 240 is energized by the electrosurgical generator operating in a vessel sealer or bipolar mode.

In accordance with the present disclosure, a distal portion of at least one of the cables 380a, 380b, 380c, 380d may include a flexible portion, while a proximal portion of the cables 380a, 380b, 380c, 380d may be rigid, such that the flexible distal portion may follow a particular path through the instrument 50. Accordingly, the biasing elements 370a, 370b, 370c, 370d may function to maintain the cables 380a, 380b, 380c, 380d in tension to prevent slack or to reduce the amount of slack in the flexible distal portion of the cables 380a, 380b, 380c, 380d. The motors 152a, 152b, 152c, 152d of the IDU 52 control the minimum tension on the cables 380a, 380b, 380c, 380d, such that the minimum tension is maintained by all four couplers 310a, 310b, 310c, 310d.

During use of the instrument 50 (e.g., when motor 152a, 152b, 152c, 152d of the IDU 52, or other powered drives, are used to rotate one or more of proximal couplers 310a, 310b, 310c, 310d), rotation of a proximal coupler 310a, 310b, 310c, 310d results in a corresponding rotation of the respective drive screw 340a, 340b, 340c, 340d. Rotation of the drive screw 340a, 340b, 340c, 340d causes longitudinal translation of the respective drive nut 350a, 350b, 350c, 350d due to the engagement between the threaded portion 341a, 341b, 341c, 341d of the drive screw 340a, 340b, 340c, 340d and the threaded aperture 351a, 351b, 351c, 351d of the drive nut 350a, 350b, 350c, 350d.

The direction of longitudinal translation of the drive nut 350a, 350b, 350c, 350d is determined by the direction of rotation of the proximal coupler 310a, 310b, 310c, 310d, which in turn, rotate the respective drive screws 340a, 340b, 340c, 340d. In embodiments, clockwise rotation of each of the drive screws 340a, 340b, 340c, 340d results in a corresponding proximal translation of cable 380a, 380b, 380c, 380d. Additionally, counterclockwise rotation of each of the drive screws 340a, 340b, 340c, 340d results in a corresponding distal translation of cables 380a, 380b, 380c, 380d.

Additionally, in one aspect, when one drive nut 350a, 350b, 350c, 350d of one of the drive assemblies 300a, 300b, 300c, 300d moves in a first longitudinal direction (e.g., proximally), it is envisioned that a different drive nut 350a, 350b, 350c, 350d from a different drive assembly 300a, 300b, 300c, 300d is moved in a second, opposite longitudinal direction (e.g., distally). Such a function may be accomplished by controlling the respective motors 152a, 152b, 152c, 152d. Such configurations function to, for example, compensate for any slack in the cables 380a, 380b, 380c, 380d or to create a slack in cables 380a, 380b, 380c, 380d. It is contemplated that each drive nut 350a, 350b, 350c, 350d may be independently driven.

Each of the motors 152a, 152b, 152c, 152d may be controlled in a corresponding manner to negate slack formation in any of cables 380a, 380b, 380c, 380d when another one of cables 380a, 380b, 380c, 380d (e.g., an opposing cable) is translated in an opposing direction. Additionally, each of the motors 152a, 152b, 152c, 152d may be controlled in a corresponding manner to create slack in any of the cables 380a, 380b, 380c, 380d when another one of cables 380a, 380b, 380c, 380d (e.g., an opposing cable) is translated in an opposing direction. Such corresponding control of the motors 152a, 152b, 152c, 152d ensures that the proximal translation of any of cables 380a, 380b, 380c, 380d is not hindered by the stationary position of an opposing cable 380a, 380b, 380c, 380d.

With reference to FIG. 6, each of the motors 152a, 152b, 152c, 152d includes a current sensor 153, a torque sensor 155, and an encoder 157. For conciseness only operation of the motor 152a is described below. The sensors 153, 155, 157 monitor the performance of the motor 152a. The current sensor 153 is configured to measure the current draw of the motor 152a and the torque sensor 155 is configured to measure motor torque. The torque sensor 155 may be any force or strain sensor including one or more strain gauges configured to convert mechanical forces and/or strain into a sensor signal indicative of the torque output by the motor 152a. The encoder 157 may be any device that provides a sensor signal indicative of the number of rotations of the motor 152a, such as a mechanical encoder or an optical encoder. Parameters which are measured and/or determined by the encoder 157 may include speed, distance, revolutions per minute, position, and the like. The sensor signals from sensors 153, 155, 157 are transmitted to the IDU controller 41d, which then controls the motors 152a, 152b, 152c, 152d based on the sensor signals. In particular, the motors 152a, 152b, 152c, 152d are controlled by an actuator controller 159, which controls torque outputted and angular velocity of the motors 152a, 152b, 152c, 152d. In embodiments, additional position sensors may also be used, which include, but are not limited to, potentiometers coupled to movable components and configured to detect travel distances, Hall Effect sensors, accelerometers, and gyroscopes. In embodiments, a single controller can perform the functionality of the IDU controller 41d and the actuator controller 159.

The IDU controller 41d provides inputs and process outputs to/from the IDU 52 during calibration and joint motion. The input signals include the desired state, desired joint angles, actual motor states, actual motor angles, actual motor current. The output signals include actual controller state, actual joint angles, desired motor state, desired motor torque, and joint limits.

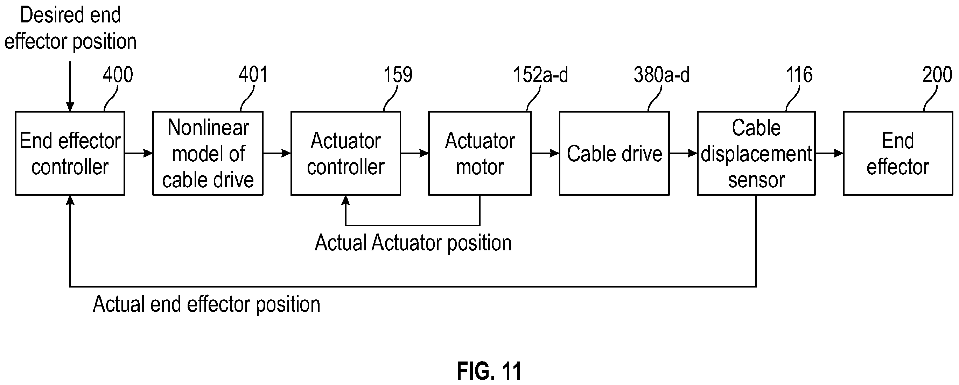

The IDU controller 41d is responsible for calibrating the instrument 50, including each of the cables 380a, 380b, 380c, 380d. With reference to FIG. 11, an end effector position controller 400 controls the movement of the needle driver 200 using an open loop control scheme in which the final position of the needle driver 200 (e.g., output) is not used in its control, rather feedback is provided by the cable displacement sensor 116 and the encoder 157. The end effector position controller 400 may be a separate controller coupled to the IDU controller 41d and may be disposed within the IDU 52 or the end effector position controller 400 may be a component of the IDU controller 41d. A desired position for the needle driver 200 is generated in response to user input through the handle controllers 38a and 38b and/or automated motion profile. The desired position is provided to the position controller 400 and is compared with the actual position of the needle driver 200 as measured by the cable displacement sensor 116. The position controller 400 computes a position error and additional control signals that are inputs to a nonlinear model 401 of the cables 380a, 380b, 380c, 380d. The model 401 determines a desired displacement of the proximal end of the cables 380a, 380b, 380c, 380d for overcoming nonlinearities such as backlash, cable stretch, stiction, and changes in length of the cable path due to bending.

Position control for instrument 50, including standard graspers, needle drivers, and bipolar instruments includes gain scheduling such that the position feedback for jaw control is disengaged when a jaw clamp is performed with a commanded input of full jaw close. This ensures that integral windup is prevented when the jaws 120 and 122 are not closed due to the object being grasped and switches the integral off until the clamp force is released and a jaw open action takes place after having transitioned from the high static friction region which results from higher jaw torques under clamp.

Position control also includes a dithering routine which maintains the instrument joints (i.e., proximal portion 112 and distal portion 114) in a dynamic friction region. As used herein, "dithering" denotes oscillating movement in opposing direction at a frequency rate of from about 40 Hz to about 150 Hz. Dithering is implemented in joint space and may be optimized based on empirical testing of a population of instruments 50 to account for instrument-to-instrument variation as well as variation over the life of the instrument 50. Joint space dithering is based on the wrist pose of the instrument 50 and configuration dependent friction. Dithering breaks away the proximal portion 112 and distal portion 114 from the static friction region and improves the controller response. Dithering also reduces the net torque needed for articulation, thereby prolonging the life of the instrument 50.

In embodiments, where the instrument 50 includes the shears 220, position control routine includes a feedforward friction model. This feedforward model reduces the controller effort of the feedback position loop and helps with controller stability and response by compensating for high friction, that is required and intrinsic to the blades design of the shears 220 jaws for adequate cutting performance.

The model 401 greatly improves the performance position control of the end effector because the model 401 compensates for the nonlinearities and allows a linear feedback controller, i.e., the actuator controller 159, to be used. Otherwise, closing the feedback loop without using the model 401 would result in a slow response, inaccuracies, and significant variation in performance as the flexible cables 380a, 380b, 380c, 380d change shape. The model 401 also compensates for variances in actual position of the jaws 120 and 122 of the surgical instrument due to the cable wrap on the pulleys 112a, 112b, 114a, 114b (FIG. 10A).

The desired displacement calculated by the model 401 is then used as an input to the actuator controller 159 which computes commanded movement commands (e.g., amount of current supplied) for each of the motors 152a, 152b, 152c, 152d. The actuator controller 159 also acts as a position controller using a feedback loop from the actual position of the motors 152a, 152b, 152c, 152d measured by the respective encoder 157. The actual motor position is compared with the desired motor position from the model 401 to further refine motor control. The motors 152a, 152b, 152c, 152d are connected to the couplers 310a, 310b, 310c, 310d that pull on the cables 380a, 380b, 380c, 380d that transmits the motion to needle driver 200. The cable displacement sensor 116 measures the actual position of each of the cables the cables 380a, 380b, 380c, 380d, which corresponds to the actual position of the needle driver 200.

In embodiments, the video feed from the endoscopic camera 51 may also be used to provide an additional control parameter to the feedback loop using vision-based algorithms, e.g., detecting movement and/or angle of the needle driver 200, and providing the visually observed angle to the position controller 400. Vision-based algorithms may be used alternatively or in addition to the feedback provided by the cable displacement sensor 116 to monitor actual position of the needle driver 200. Additionally, measured cable tension may also be used as an additional input to the position controller 400. Measured torque may be used to estimate applied forces and torque on the tissue by the needle driver 200.

In further embodiments, the model 401 may be an adaptive nonlinear model of the cables 380a, 380b, 380c, 380d that automatically adjusts control parameters to compensate for observable parameters that slowly change over time, such as overall curvature changes of the flexible cable conduit. A neural network or other artificial intelligence-based algorithms may be used to develop and implement the nonlinear model 401. The model 401 may be taught based on a data set of commanded motions and observed (i.e., measured) positions of the needle driver 200.

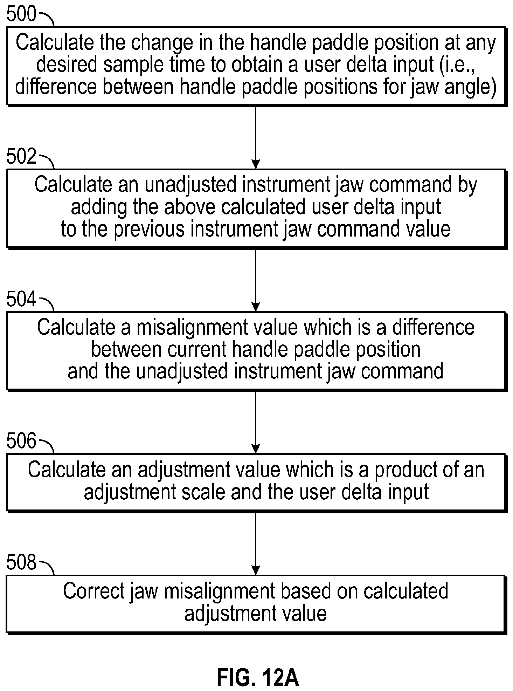

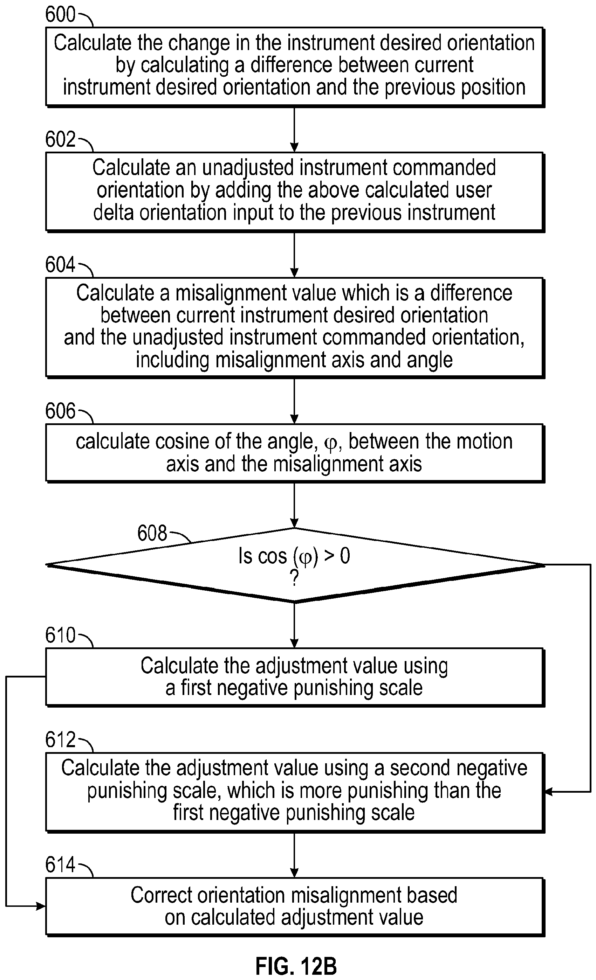

With reference to FIGS. 12A and 12B, a realignment algorithm is shown in the flow charts. The realignment algorithm is embodied as software instructions executed by the controller 21a or any other suitable controller of the surgical robotic system 10 and is configured to adjust the delta orientation and delta open/close commands of the jaws 120 and 122 in order to reduce the misalignment between the desired inputs and the commanded outputs.

With reference to FIG. 12A, opening and closing of the jaws 120 and 122 is performed in response to an instrument jaw command, which is based on the input from the handle controller 38a. The instrument jaw command is a value representing the ratio of the commanded opening angle of the opposing jaws 120 and 122 to the maximum opening angle of the opposing jaws 120 and 122 that can be commanded. The instrument jaw command may be from 0 to 1, with 0 indicating a fully closed state and 1 indicating a fully opened state. Thus, the handle controller 38a outputs the instrument jaw command and the jaws 120 and 122 are commanded to attain the position based on that command.

Similarly, the handle paddle position of the handle controller 38a is a value representing actual opening ratio of the paddle, i.e., the ratio of the current opening angle of the paddle to the maximum possible opening angle of the paddle. The handle paddle position may be from 0 to 1, with 0 indicating a fully closed state and 1 indicating a fully opened state.

Jaw misalignment is defined as the scalar difference between the handle paddle position and instrument jaw command at any given sample time. Thus, the misalignment is calculated by the controller 21a between the paddle position and the instrument command, without factoring in any tracking error that may be due to the actual jaw position not matching the instrument jaw command.

At step 500, the controller 21a continuously calculates the change in the handle paddle position at any desired sample time (e.g., k). In particular, the controller 21a calculates a difference between current handle paddle position and the previous (i.e., at sample time k-1) handle paddle position. The difference between handle paddle positions is a user delta input and represents the amount of motion provided by the user with respect to the previous sample time.

At step 502, the controller 21a also calculates an unadjusted instrument jaw command by adding the above calculated user delta input to the previous instrument jaw command (the instrument jaw command sent out at time k-1). The unadjusted instrument jaw command represents what the current instrument jaw command would be solely based on the user delta input, disregarding any realignment efforts. In other words, this command would move the jaws in a way that would keep the initial misalignment present in the system rather than reducing it (until range of motion limits are hit).

At step 504, the controller 21a calculates a misalignment value which is a difference between current handle paddle position and the unadjusted instrument jaw command and represents the amount of misalignment in the outgoing jaw command without any realignment.

At step 506, the controller 21a calculates an adjustment value which is a product of an adjustment scale factor and the user delta input. The adjustment scale factor is a positive scalar if the user delta input and the misalignment value agree in sign (i.e., both are positive or negative) and is a negative scalar otherwise (i.e., one is negative and another is positive). To correct for misalignment, at step 508, the controller 21a then adds the adjustment value to the unadjusted instrument jaw command. In addition, the absolute value of the adjustment value is compared to the absolute value of the misalignment value to confirm that the absolute adjustment value does not exceed the absolute misalignment value to prevent overcorrection.

With reference to FIG. 12B, orientation of the instrument 50 is performed in response to an instrument commanded orientation, which is based on the input from the handle controller 38a. Instrument commanded orientation is a first rotation matrix (e.g., 3x3 matrix) provided by the handle controller 38a that the tool center point (TCP) of the instrument 50 (i.e. the coordinate frame of the needle driver 200) is commanded to attain with respect to a base frame (i.e., base frame of the robotic arm 40).

Instrument desired orientation is represented as a second rotation matrix (e.g., 3x3 matrix) that represents the "aligned" orientation of the TCP of the instrument 50 with respect to the base frame of the robotic arm 40. This orientation is derived from the orientation of the handle controller 38a and a hand-eye coordination mapping. When instrument desired orientation is represented in endoscope feed frame through proper transformations, the instrument orientation matches the handle orientation as represented in the surgeon frame. The surgeon frame and the endoscope feed frame are the two frames with respect to which hand-eye coordination is defined. In other words, for correct visual correspondence, the translations of the handle controller 38a on the x (y or z) axis of the surgeon frame should result in translations of the instrument 50 on the x (y or z) axis of the endoscope feed frame. A scaled handle orientation may also be used to define the instrument desired orientation instead of the actual handle orientation.

Orientation misalignment is defined as a difference between instrument commanded orientation and instrument desired orientation at any given sample time. Orientation misalignment is calculated by the controller 21a between the desired orientation and the commanded orientation, without factoring in any tracking error that may be due to the actual instrument orientation not perfectly tracking the instrument commanded orientation. The difference between first and second rotation matrices is obtained by first computing the transpose of the second matrix, then post-multiplying the first matrix and then converting the result into "axis-angle" representation. The resulting axis is represented in the frame of the second rotation matrix.

At step 600, the controller 21a continuously calculates the change in the instrument desired orientation at any desired sample time (e.g., k). In particular, the controller 21a calculates a difference between current instrument desired orientation and the previous (i.e. at sample time k-1) instrument desired orientation. The difference between instrument desired orientations is a user delta orientation input and represents the amount of motion provided by the user with respect to the previous sample time. The difference is converted to axis-angle representation, i.e., a motion axis, which is 3x1 vector. The angle of orientation defines a motion angle and it is a positive scalar.

At step 602, the controller 21a also calculates an unadjusted instrument commanded orientation by adding the above calculated user delta orientation input to the previous instrument commanded orientation (i.e., the instrument orientation command sent out at time k-1). This is done using proper frames when rotation matrix to axis-angle transitions are made. This represents what the current instrument commanded orientation would be solely based on the user orientation input, disregarding any realignment efforts. In other words, this command would move the instrument orientation in a way that would keep the initial misalignment present in the system rather than reducing the misalignment (until range of motion limits are hit).

At step 604, the controller 21a further calculates a misalignment value which is a difference between current instrument desired orientation and the unadjusted instrument commanded orientation. The misalignment value represents the amount of misalignment in the outgoing instrument orientation command without any efforts to realign the instrument 50. The misalignment value is also converted to axis-angle format. The axis of the difference between current instrument desired orientation and the unadjusted instrument commanded orientation is the misalignment axis, which is a 3x1 vector. Similarly, the angle of the difference is misalignment angle and is a positive scalar.

At step 606, the controller 21a calculates the cosine of the angle, $\phi$, between the motion axis and the misalignment axis, when both vectors are represented in a common frame, e.g., base frame of the robotic arm 40. The controller 21a also calculates an adjusted motion angle by using the calculated cosine (e.g., cos($\phi$)), an adjustment scale and the motion angle using the formula (I) below:

$$\text{adjusted motion angle} = (\text{motion angle}) + \text{abs}(\cos(\phi)) \text{ x (adjustment scale) x (motion angle)}.$$

At step 608, the controller determines if cos($\phi$) > 0. If cos($\phi$) > 0, at step 610, the controller 21a selects a first negative punish scale, i.e., factor. If cos($\phi$) < 0, at step 612, the controller 21a selects a second negative punish scale, i.e., factor, larger than the first one in absolute value. At step 614, the controller 21a corrects orientation misalignment based on the calculated adjustment value in steps 610 or 612. The controller 21a also caps the motion angle adjustment (i.e., the difference between the motion angle and the adjusted motion angle) so that its absolute value does not exceed misalignment angle at any given time. The controller 21a further calculates the adjusted instrument commanded orientation by using the adjusted motion angle, the same motion axis and the previously instrument commanded orientation. The realignment algorithm does not change the axis of commanded motion, but adjusts the amount of motion along that axis.

The alignment algorithm may be modified based on the type of the end effector being used, e.g., needle grasper 200, stapler 215, shears 220, electrocautery blade 230, vessel sealer 240, etc. Upon coupling the instrument 50 to the IDU 52, the controller 21a is configured to determine the type of the instrument 50 and/or end effector by accessing a memory of the instrument 50, which may store various operating parameters pertaining to the instrument 50. The stored parameters may be used by a software module executed by the controller 21a to control the instrument 50 (e.g., kinematic characteristics) as well as modifications to the alignment algorithm. The modifications may be stored locally by the system 10, in the memory of the instrument 50, or remotely (e.g., server) and may be retrievable by the controller 21a upon identification of the instrument 50 and/or end effector.

The desired instrument or end effector orientation may be based on the type of the end effector and specific functionality, ignoring specific input from the handle controller 38a. For example, with the stapler 215, realignment may be made to the position of the stapler 215 when the stapler 215 is in a so-called "armed" state, i.e., ready to eject staples, rather than the standard orientation based on orientation of hand-eye and hand controllers 38a / 38b.

Conversely, realignment may be based not only on the orientation of the hand controllers 38a / 38b but also take into consideration the surgical task being performed. Other components of the system 10 may be used by the realignment algorithm, such as image processing of real-time video of the end effector captured by the camera 51. Thus, in addition to the sensors of the IDU 52 and the instrument 50, image processing may be used to determine misalignment.

In addition, the controller 21a may also adjust the realignment algorithm based on the particular surgical task being performed. The task may be determined using machine learning techniques processing tasks or steps previously performed during the procedure. Thus, realignment speed may be adjusted based on the task. In embodiments, realignment may be performed at a faster rate when retracting or moving organs around and may be slower for delicate tasks like dissecting near critical structures. Furthermore, the realignment may be based on location of the end effector being near the edge of the orientation workspace. The alignment may be configured to slowly drift the aligned angle so that the handle orientation required is more comfortable.

In embodiments, the rate of convergence, namely, the rate at which misalignment is corrected, may be also specific to the type of end effector being used, to account for differences in dimensions of the jaws, such as length. With respect to longer jaw members, such as those of the stapler 215, which may be about 50 mm, the rate of convergence may be reduced. Whereas for shorter jaw instruments, like the needle driver 200, the rate of convergence may be increased.

The realignment algorithm may be also executed selectively, based on a region of misalignment, such that misalignment is only corrected when misalignment occurs in a certain set region and is not adjusted when it occurs outside the set region. A "region" as used herein refers to a position of one or both of the jaw members and/or the angle therebetween. The misalignment region may also be based on the type of instrument 50 being used. For precisely operated instruments, such as the stapler 215 and the vessel sealer 240, the misalignment region may be small, and cover more movement of the end effector that is adjusted for misalignment. For less precisely operated instruments, such as graspers, retraction tools, etc., the misalignment region may be large, to cover less movement of the end effector.

In addition to whether the misalignment is performed based on the region, the degree of correction for misalignment may also be based on the region. In particular, multiple regions of misalignment may have a different punish factor, associated therewith. Similar to step 608 of FIG. 12A, the punish factor may be modified throughout the misalignment adjustment. Thus, the factor is increased when the misalignment is large and then reduced when approaching a smaller level of misalignment, e.g., transitioning from step 612 to 610.

These regions and punish factors may be instrument dependent. In particular, when using the stapler 215, it may be desirable to realign quickly for larger misalignments and then slow down once misalignment is reduced. This shift in rate of alignment matches manual instrument use, i.e., a surgeon might use an instrument to do a quick movement of the handle to get basic realignment in place and then to provide for more precise control once broader alignment is complete.

For some instruments with fewer degrees of freedom in a wrist, such as the stapler 215, which only includes a single articulating axis, the realignment algorithm may be applied only to the available degrees of freedom, e.g., roll and pitch for the stapler 215 and for the electrocautery blade 230 only pitch and yaw.

Similar to orientation realignment, the jaw opening angle realignment may be changed at specific rates based on instrument type. Thus, realignment may be done faster when opening than closing, or realignment may be only implemented in only one direction (i.e., either opening or closing).

It will be understood that various modifications may be made to the embodiments disclosed herein. In embodiments, the sensors may be disposed on any suitable portion of the robotic arm. Therefore, the above description should not be construed as limiting, but merely as exemplifications of various embodiments. Those skilled in the art will envision other modifications within the scope of the claims appended thereto.

**Claims**

1. A surgical robotic system (10) comprising:

       a surgical console (30) including a handle controller (38a);
       a robotic arm (40) including an instrument (50) having:

a plurality of cables (380a,b,c) that are movable longitudinally; and
an end effector movable (200) by the cables, the end effector having a pair of opposing jaws; and

a processor configured to:

determine an amount of orientation misalignment of an instrument tool center point, and
adjust the instrument based on the amount of orientation misalignment of the instrument tool center point;

wherein the processor is further configured to calculate a motion axis based on a difference between a current instrument desired orientation and a previous instrument desired orientation, the current instrument desired orientation being based on an orientation of the handle controller; wherein the processor is further configured to calculate a user delta orientation input based on a difference between a current orientation of the handle controller and a previous orientation of the handle controller; wherein the processor is further configured to calculate an unadjusted instrument commanded orientation by adding the user delta orientation input to a previous instrument commanded orientation; wherein the processor is further configured to calculate a misalignment axis, which is a difference between the current instrument desired orientation and the unadjusted instrument commanded orientation; wherein the processor is further configured to calculate an adjustment value based on an angle between the motion axis and the misalignment axis, characterized wherein the processor is further configured to calculate the adjustment value using a first negative punishing scale in response to a cosine of the angle being positive; wherein the processor is further configured to calculate the adjustment value using a second negative punishing scale in response to a cosine of the angle being negative, the second negative punishing scale being larger than the first negative punishing scale in absolute value.

**Patentansprüche**

1. Chirurgisches Robotersystem (10), umfassend:

eine chirurgische Konsole (30), die eine Griff-Steuerung (38a) einschließt;
einen Roboterarm (40), der ein Instrument (50) einschließt, das aufweist:

eine Vielzahl von Kabeln (380a, b, c), die in Längsrichtung beweglich sind; und
einen Endeffektor, der durch die Kabel beweglich (200) ist, wobei der Endeffektor ein Paar gegenüberliegender Backen aufweist; und
einen Prozessor, der konfiguriert ist zum:

Bestimmen eines Grads einer Orientierungsfehlausrichtung eines Instrumentenwerkzeugmittelpunkts und
Anpassen des Instruments basierend auf dem Grad der Orientierungsfehlausrichtung des Instrumentenwerkzeugmittelpunkts;
wobei der Prozessor ferner konfiguriert ist, um eine Bewegungsachse basierend auf einer Differenz zwischen einer aktuellen gewünschten Instrumentenorientierung und einer vorherigen gewünschten Instrumentenorientierung zu berechnen, wobei die aktuelle gewünschte Instrumentenorientierung auf einer Orientierung der Griff-Steuerung basiert; wobei der Prozessor ferner konfiguriert ist, um eine Benutzer-Delta-Orientierungseingabe basierend auf einer Differenz zwischen einer aktuellen Orientierung der Griff-Steuerung und einer vorherigen Orientierung der Griff-Steuerung zu berechnen; wobei der Prozessor ferner konfiguriert ist, um durch Addieren der Benutzer-Delta-Orientierungseingabe zu einer vorherigen von dem Instrument angeforderten Orientierung eine nicht angepasste, von dem Instrument angeforderte Orientierung zu berechnen; wobei der Prozessor ferner konfiguriert ist, um eine Fehlausrichtungsachse zu berechnen, die eine Differenz zwischen der aktuell gewünschten Instrumentenorientierung und der nicht angepassten, von dem Instrument angeforderten Orientierung ist; wobei der Prozessor ferner konfiguriert ist, um einen Anpassungswert basierend auf einem Winkel zwischen der Bewegungsachse und der Fehlausrichtungsachse zu berechnen, wobei **dadurch gekennzeichnet, dass** der Prozessor ferner konfiguriert ist, um den Anpassungswert unter Verwendung einer ersten negativen Strafskala als Reaktion darauf zu berechnen, dass ein Kosinus des Winkels positiv ist; wobei der Prozessor ferner konfiguriert ist, um den Anpassungswert unter Verwendung einer zweiten negativen Strafskala als Reaktion darauf zu berechnen, dass ein Kosinus des Winkels negativ ist, wobei die zweite negative Strafskala in einem Absolutwert größer ist als die erste negative Strafskala.

**Revendications**

1. Système robotique chirurgical (10) comprenant :

   une console chirurgicale (30) comportant un dispositif de commande de poignée (38a) ;
   un bras robotique (40) comportant un instrument (50) ayant :

   une pluralité de câbles (380a,b,c) qui sont mobiles longitudinalement ; et
   un effecteur terminal mobile (200) par les câbles, l'effecteur terminal ayant une paire de mâchoires opposées ; et
   un processeur configuré pour :

   déterminer une quantité de désalignement d'orientation d'un point central d'outil d'instrument, et
   ajuster l'instrument en fonction de la quantité de désalignement d'orientation du point central d'outil d'instrument ;
   dans lequel le processeur est en outre configuré pour calculer un axe de mouvement en fonction d'une différence entre une orientation actuelle de l'instrument souhaitée et une orientation précédente de l'instrument souhaitée, l'orientation actuelle de l'instrument souhaitée en fonction d'une orientation du dispositif de commande de poignée ; dans lequel le processeur est en outre configuré pour calculer une entrée d'orientation delta de l'utilisateur en fonction d'une différence entre une orientation actuelle du dispositif de commande de poignée et une orientation précédente du dispositif de commande de poignée ; dans lequel le processeur est en outre configuré pour calculer une orientation non ajustée commandée de l'instrument en ajoutant l'entrée d'orientation delta de l'utilisateur à une orientation précédente commandée de l'instrument ; dans lequel le processeur est en outre configuré pour calculer un axe de désalignement, qui est une différence entre l'orientation actuelle de l'instrument souhaitée et l'orientation non ajustée commandée de l'instrument ; dans lequel le processeur est en outre configuré pour calculer une valeur d'ajustement en fonction d'un angle entre l'axe de mouvement et l'axe de désalignement, **caractérisé en ce que** le processeur est en outre configuré pour calculer la valeur d'ajustement à l'aide d'une première échelle de punition négative en réponse au fait qu'un cosinus de l'angle est positif ; dans lequel le processeur est en outre configuré pour calculer la valeur d'ajustement à l'aide d'une seconde échelle de punition négative en réponse au fait qu'un cosinus de l'angle est négatif, la seconde échelle de punition négative étant plus grande que la première échelle de punition négative en valeur absolue.

FIG. 1

FIG. 2

**FIG. 3**

**FIG. 4**

**FIG. 5**

**FIG. 6**

**FIG. 7**

FIG. 8

EP 4 304 509 B1

FIG. 9A

FIG. 9B

FIG. 10A

**FIG. 10B**

**FIG. 10C**

**FIG. 10D**

**FIG. 10E**

Desired end effector position

End effector controller **400** → Nonlinear model of cable drive **401** → Actuator controller **159** → Actuator motor **152a-d** → Cable drive **380a-d** → Cable displacement sensor **116** → End effector **200**

Actual Actuator position

Actual end effector position

FIG. 11

500 ⌐
Calculate the change in the handle paddle position at any
desired sample time to obtain a user delta input (i.e.,
difference between handle paddle positions for jaw angle)

502 ⌐
Calculate an unadjusted instrument jaw command by
adding the above calculated user delta input
to the previous instrument jaw command value

504 ⌐
Calculate a misalignment value which is a difference
between current handle paddle position
and the unadjusted instrument jaw command

506 ⌐
Calculate an adjustment value which is a product of an
adjustment scale and the user delta input

508 ⌐
Correct jaw misalignment based on calculated
adjustment value

**FIG. 12A**

600

Calculate the change in the instrument desired orientation by calculating a difference between current instrument desired orientation and the previous position

602

Calculate an unadjusted instrument commanded orientation by adding the above calculated user delta orientation input to the previous instrument

604

Calculate a misalignment value which is a difference between current instrument desired orientation and the unadjusted instrument commanded orientation, including misalignment axis and angle

606

calculate cosine of the angle, φ, between the motion axis and the misalignment axis

608

Is cos (φ) > 0 ?

610

Calculate the adjustment value using a first negative punishing scale

612

Calculate the adjustment value using a second negative punishing scale, which is more punishing than the first negative punishing scale

614

Correct orientation misalignment based on calculated adjustment value

FIG. 12B

**EP 4 304 509 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63159559
- US 2021045826 A
- WO 2020118149 A